# EUROPEAN PATENT SPECIFICATION

(11) **EP 1 062 348 B1**
(45) Date of publication and mention of the grant of the patent: **15.06.2005**
(21) Application number: 99911005.9
(22) Date of filing: 11.03.1999
(51) Int. Cl.: C12N 15/55, C12N 9/22, C12N 15/62, C07K 19/00, C07K 16/46, A61K 38/46

(54) **RECOMBINANT ONCONASE AND FUSION PROTEINS OF RECOMBINANT ONCONASE**
REKOMBINANTE ONCONASE UND FUSIONSPROTEINE DAVON
ONCONASE RECOMBINANTE ET PROTEINES DE FUSION DE L'ONCONASE RECOMBINANTE

(30) Priority: 11.03.1998 US 77557 P
(43) Date of publication of application: 27.12.2000
(60) Divisional application: 04077093.5
(73) Proprietor: Immunomedics, Inc., Morris Plains, New Jersey 07950 (US)
(72) Inventor: GOLDENBERG, David, M., Mendham, NJ 07945 (US); HANSEN, Hans, Little Egg Harbor, NJ 08087 (US); LEUNG, Shui-on, Madison, NJ 07940 (US)
(74) Representative: Maschio, Antonio, Dr.
(86) International application number: PCT/US1999/004252
(87) International publication number: WO 1999/046389

(56) References cited:
- WO-A-97/31116
- WO-A-97/38112
- WO-A-98/50435
- WOJCIECH ARDELT ET AL.: "Amino acid sequence of an anti-tumor protein from Rana pipiens oocytes and early embryos " JOURNAL OF BIOLOGICAL CHEMISTRY, vol. 266, no. 1, 5 January 1991 (1991-01-05), pages 245-251, XP000172527 MD US
- ESTER BOIX ET AL.: "Role of the N terminus in RNase A homologues: differences in catalytic activity, ribonuclease inhibitor interaction and cytotoxicity" JOURNAL OF MOLECULAR BIOLOGY, vol. 257, no. 5, 19 April 1996 (1996-04-19), pages 992-1007, XP000675661
- LEUNG, S.O. ET AL. : "Recombinant expression of ribonuclease of Rana pipien origin" PROCEEDINGS OF THE AMERICAN ASSOCIATION FOR CANCER RESEARCH, vol. 40, March 1999 (1999-03), pages 353-354, XP002105912

## Description

### Background of the Invention

The present invention relates to recombinantly-produced Onconase molecules and fusion proteins containing the same.

Onconase is a non-mammalian ribonuclease (RNAse) with a molecular weight of 12,000 that is purified from *Rana pipiens* oocytes and early embryos. Onconase causes potent inhibition of protein synthesis in the rabbit reticulocyte lysate (IC₅₀ 10⁻¹¹ M) and when microinjected into *Xenopus* oocytes (IC₅₀ 10⁻¹⁰ M). Unlike other members of the RNase A superfamily, Onconase does not degrade oocyte rRNA. Upon binding to the cell surface receptors of sensitive cells and its cytosolic internalization, Onconase causes cell death as a result of potent protein synthesis inhibition by a mechanism involving inactivation of cellular RNA. Onconase is not inhibited by mammalian placental ribonuclease inhibitor and this may explain Onconase's enhanced cytotoxicity when compared to the mammalian enzymes.

Animal toxicology studies show that Onconase displays a predictable, dose-dependent and reversible toxicity in both rats (dose range 0.01-0.02 mg/kg) and dogs (0.005-0.15 mg/kg). Mice inoculated with the aggressive M109 Madison lung carcinoma and treated with both daily and weekly schedule of intraperitoneally-administered Onconase, showed significantly prolonged survival. Most striking results were seen in a group of mice treated with a weekly schedule of Onconase in which six of eighteen animals survived long-term and were apparently cured of cancer.

Onconase has been shown in clinical trials to have anti-tumor activity against a variety of solid tumors. In this regard it has been used both alone and combined with other anti-tumor agents such as tamoxifen, *e.g*., when treating patients with pancreatic cancer. When used as an anti-tumor agent, Onconase can be conjugated to a marker which targets it to a specific cell type.

In a Phase I study, patients suffering from a variety of relapsing and resistant tumors were treated intravenously with Onconase. A dose of 60-690 µ/m² of Onconase resulted in the possible side effects of flushing myalgias, transient dizziness, and decreased appetite in general. The observed toxicities, including the dose-limiting renal toxicity manifested by increasing proteinuria, peripheral edema, azotemia, a decreased creatinine clearance, as well as fatigue, were dose-dependent and reversible, which is in agreement with the animal toxicology studies. No clinical manifestations of a true immunological sensitization was evident, even after repeated weekly intravenous doses of Onconase. The maximum tolerated dose, mainly due to renal toxicity, was found to be 960 µg/m². There were also some objective responses in non-small cell lung, esophageal, and colorectal carcinomas. Nevertheless, Onconase was well-tolerated by animals and the majority of human patients tested, demonstrated a consistent and reversible clinical toxicity pattern, and did not induce most of the toxicities associated with most of the chemotherapeutic agents, such as myelosuppression and alopecia.

Onconase thus has many desirable characteristics, including small size, animal origin, and anti-tumor effects *in vitro* and *in vivo*. It is well-tolerated and refractory to human RNase inhibitors. However, Onconase purified from *Rana pipiens* oocytes has undesirable properties. The fact that it is obtained from a natural source makes it more difficult and expensive to obtain sufficient quantities. Since it is not derived from humans, or even mammals, it typically stimulates undesirable immune responses in humans. Accordingly, it would be advantageous recombinantly to produce native Onconase which retains the cytotoxic properties of Onconase purified from *Rana pipiens* oocytes, but does not have the undesirable immune responses in humans.

Attempts to produce native Onconase in *E. coli* by recombinant DNA methodology have failed. Onconase has an N-terminal pyroglutamyl residues which is required for proper folding of the molecule. This residue forms part of the phosphate binding pocket of Onconase, and is essential for RNAse and anti-tumor activity. The initiation codon in *E. coli* inserts N-formyl-methionine in peptides as the N-terminal amino acid residue. Therefore, native Onconase recombinantly-produced in *E. coli* does not have pyroglutamyl as the N-terminal residue.

WO 97/31116 claims to have solved the problem of producing a modified Onconase that retains cytotoxic activity. It discloses a recombinant ribonuclease that has an amino terminal end beginning with a methionine followed by an amino acid other than glutamic acid, a cysteine at positions 26, 40, 58, 84, 95 and 110, a lysine at position 41, and a histidine at position 119 of bovine RNAse A, and a native Onconase-derived amino acid sequence. WO 97/3116 does not recognize the importance of pyroglutamate as the N-terminal residue, and does not produce an Onconase molecule with an N-terminal pyroglutamate. To the contrary, WO 97/3116 suggests the addition of amino terminal sequences and/or fusion at the N-terminus to a ligand molecule.

### Brief Description of the Drawing

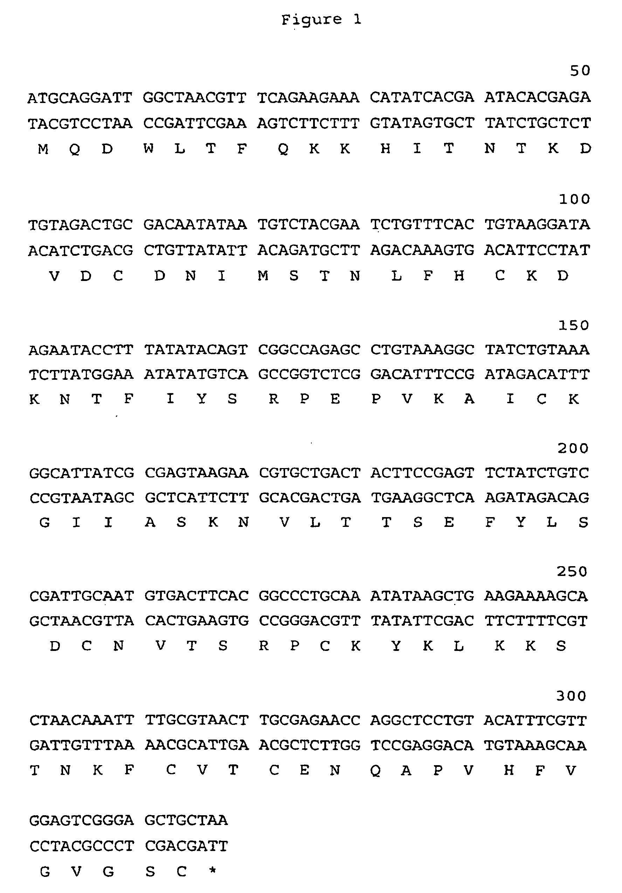

Figure 1 shows the nucleic acid sequence and amino acid sequence of NfM-Onconase.

### Summary of the Invention

It is an object of the present invention to provide a recombinantly-produced Onconase, which retains the cytotoxic properties of Onconase, while eliminating the undesirable side effects.

These and other objects according to the invention are provided by an Onconase LL2 scFv molecule which has pyroglutamate as the N-terminal residue, wherein said Onconase molecule is recombinantly-produced in -scFv, wherein said RNAse includes pyroglutamate as the N-terminal residue and said scFv is an LL2 scFv. *E. coli*. The recombinantly-produced Onconase molecule has the sequence and structure of Onconase purified from *Rana pipiens.*

The fusion protein may be made by recombinantly expressing a nucleic acid sequence encoding Onconase and a targeting moiety. The targeting moiety is an antibody fragment, which is scFv.

A composition comprising recombinantly-produced Onconase fusion protein according to the invention and a pharmaceutically acceptable carrier is provided. The compositions are useful in a method of treatment for lymphoms, which comprises administering to a subject in need of such treatment a therapeutically effective amount of the recombinantly-produced Onconase, fusion protein, according to the invention.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Description of Preferred Embodiments

It surprisingly has been discovered that an Onconase molecule which (1) has the sequence of native Onconase, (2) retains the proper folding of native Onconase and (3) has cytotoxic activity similar to that of Onconase purified from oocytes of *Rana pipiens*, can produced recombinantly in *E*. *coli*. In accordance with the present invention, the cDNA coding for Onconase is extended by one triplet which codes for N-formyl-methionine. When expressed recombinantly, the mutant Onconase has N-formyl-methionine as the N-terminal amino acid, and glutaminyl as the penultimate N-terminal residue. The expression product produced in accordance with the present invention is referred to herein as NfM-onconase. Following expression, the N-formyl methionine residue is cleaved and the penultimate glutaminyl residues is cyclized to produce Onconase with an N-terminal pyroglutamate residue, referred to herein as rOnconase. rOnconase has the same structure and function as native Onconase.

### Definitions

Unless otherwise defined, all technical and scientific terms used have the same meaning as commonly understood by one of ordinary skill in the art. For purposes of the present invention, the following terms are defined as follows:
Amino acids are referred to by name or by either their commonly known three-letter symbols or by the one-letter IUPAC symbols. Nucleotides are referred to by their commonly accepted single-letter codes.
"Conservatively modified variations" of a particular nucleic acid sequence refer to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given polypeptide. For instance, the codons GCA, GCC, GCG and GCU all encode the amino acid alanine. Thus, at every position where an alanine is specified by a codon, the codon can be altered to any of the corresponding codons described without altering the encoded polypeptide. Each codon in a nucleic acid except AUG which encodes methionine can be modified to yield a functionally identical molecule. The nucleic acid sequences described herein also encompass these alterations.
"Conservatively modified variations" of an amino acid sequence include individual substitutions which alter a single amino acid or a small percentage of amino acids in an encoded sequence, where the alterations result in the substitution of an amino acid with a chemically similar amino acid. Conservative substitutions are well known to those of skill in the art. The following six groups each contain amino acids that are conservative substitutions for one another:
   1. Alanine, Serine, Threonine
   2. Aspartic acid, Glutamic Acid
   3. Asparagine, Glutamine
   4. Arginine, Lysine
   5. Isoleucine, Leucine, Methionine, Valine, and
   6. Phenylalanine, Tyrosine, Tryptophan.
   "Conservatively modified variations" of an amino acid sequence also include deletions or additions of a single amino acid or a small percentage of amino acids in an encoded sequence,
   where the additions and deletions result in the substitution of an amino acid with a chemically similar amino acid. The amino acid sequences described herein also encompass these variations.

The terms "isolated" or "biologically pure" refer to material which is substantially or essentially free from components which normally accompany it as found in its naturally occurring environment. The isolated material optionally comprises material not found with the material in its natural environment.

The term "nucleic acid" refers to a deoxyribonuclease or ribonucleotide polymer in either single- or double-stranded form and, unless otherwise limited, encompasses known analogues of natural nucleotides that hybridize to nucleic acids in a manner similar to naturally occurring nucleotides. Unless otherwise indicated, a particular nucleic acid sequence includes its complementary sequence.

An "expression vector" includes a recombinant expression cassette which includes a nucleic acid which encodes a polypeptide according to the invention which can be transcribed and translated by a cell. A recombinant expression cassette is a nucleic acid construct, generated recombinantly or synthetically, with a series of specified nucleic acid elements which permit transcription of a particular nucleic acid in a target cell. The expression vector can be part of a plasmid, virus, or nucleic acid fragment. Typically, the recombinant expression cassette portion of the expression vector includes a nucleic acid to be transcribed and a promoter operably linked thereto.

The term "recombinant" when used with reference to a protein indicates that a cell expresses a peptide or protein encoded by a nucleic acid whose origin is exogenous to the cell. Recombinant cells can express genes that are not found within the native (non-recombinant) form of the cell. Recombinant cells also can express genes found in the native form of the cell wherein the genes are re-introduced into the cell by artificial means, for example, under the control of a heterologous promoter.

The term "substantial identity" or "substantial similarity" in the context of a polypeptide indicates that a polypeptide comprises a sequence with at least 80%, more preferably 90%, and most preferably at least 95% identity with a reference sequence. Two polypeptides that are substantially identical means the one of the polypeptides is immunologically reactive with antibodies raised against the second peptide. Two nucleic acids are substantially identical is the two molecules hybridize to each other under stringent conditions. Generally, stringent conditions are selected to be about 5°C to 20°C lower than the thermal melting point (Tₘ) for a specific sequence at a defined ionic strength and pH. The Tₘ is the temperature (under defined ionic strength and pH) at which 50% of the target sequence hybridizes to a perfectly matched probe. However, nucleic acids which do not hybridize to each other under stringent conditions are still substantially identical if the polypeptides they encode are substantially identical.

An "antibody" includes both whole antibodies and antibody fragments such as F(ab')₂, F(ab)₂, Fab', Fab, Fv and the like, including hybrid fragments. Also useful are any subfragments that retain the hypervariable, antigen-binding region of an immunoglobulin.

A "targeting moiety" is an antibody, cytokine or growth factor that is specific to a marker on a given cell type. A targeting moiety can be used to specifically deliver an attached molecule to a given cell type, by preferentially associating with the marker associated with that cell type.

A "fusion protein" is a chimeric molecule formed by joining two or more polypeptides, more particularly, Onconase and a targeting moiety. Onconase and the targeting moiety are joined through a peptide bond formed between the amino terminus of the targeting moiety and the carboxyl terminus of Onconase, and are expressed recombinantly by a nucleic acid sequence encoding the fusion protein. A single chain fusion protein is a fusion protein that has a single contiguous polypeptide backbone.

A "chemical conjugate" is a conjugate formed by the chemical coupling of Onconase and a targeting moiety.

"A pharmaceutically acceptable carrier" is a material that can be used as a vehicle for administering the Onconase or fusion protein because the material is inert or otherwise medically acceptable, as well as compatible with the fusion protein or armed ligand.

In accordance with the present invention, nucleic acid that encodes native Onconase may be prepared by cloning and restriction of appropriate sequences, or using DNA amplification with polymerase chain reaction (PCR). The amino acid sequence of Onconase can be obtained from Ardelt *et al., J. Biol. Chem.,* **256**: 245 (1991), and cDNA sequences encoding native Onconase, or a conservatively modified variation thereof, can be gene-synthesized by methods similar to the *en bloc* V-gene assembly in hLL2 humanization. Leung et *al*., *Mol*. *Immunol.,* **32**: 1413 (1995). For expression in *E. coli*, a translation initiation codon ATG, is placed in-frame preceding the Onconase cDNA sequence. The translated protein then contains an additional Met at the -1 position.

Alternatively, nucleic acid that encodes native Onconase may be synthesized *in vitro.* Chemical synthesis produces a single-stranded oligonucleotide. This may be converted to a double-stranded DNA by hybridization with a complementary sequence, or by polymerization with a DNA polymerase using the single strand as a template. While chemical synthesis is limited to sequences of about 100 bases, longer sequences may be obtained by ligating shorter sequences.

As noted, a gene encoding native Onconase, or a conservatively modified variation thereof, is modified to include a codon for N-formyl-methionine at the N-terminus. The thus-obtained NfM-onconase gene is operably linked to a suitable *E*. *coli* promoter, such as the T7, trp, or lambda promoter, and inserted in an expression cassette. Preferably a ribosome binding site and transcription termination signal also are included in the expression cassette. An expression vector that contains the cassette is transferred into an *E. coli* expression host by methods known to those of skill in the art. Transformed cells can be selected resistance to antibiotics conferred by marker genes contained in the expression vector.

The transformed *E. coli* host expresses NfM-Onconase, -LL2-scFv which may be contained in an inclusion body. Although Onconase possesses potent RNase activity, NfM-Onconase does not. This is because the N-terminal pyroglutamate on Onconase is part of the active site, as demonstrated by the crystal structure of Onconase. The inherent nature of the bacterial expression system, which requires an N-terminal Met, means that the bacterial expression product is inactive. This enables recombinant expression of NfM-Onconase -LL2-scFv in bacterial expression systems. While NfM-Onconase is not toxic, it also may be expressed as inactive inclusion bodies.

NfM-Onconase LL2-scFv can be isolated and purified according to standard procedures, including ammonium sulfate precipitation, affinity columns, column chromatography, and gel electrophoresis. Substantially pure compositions of at least about 90-95% homogeneity, and preferably 98-99% homogeneity, are preferred.

Following purification of the NfM-onconase, and refolding of the molecule if it was expressed in an inclusion body, the N-formyl-methionine is removed by digestion with aminopeptidase. A suitable aminopeptidase is *Aeromonas* aminopeptidase, as disclosed in Shapiro *et al*. *Anal. Biochem.* **175**:450-461 (1988). Incubation of the resulting product results in spontaneous cyclization of the N-terminal glutamine residue, to form a molecule having the structure and function of native Onconase.

The recombinantly-produced Onconase can be used as an alternative or complement to existing toxins.

An exemplary antigen for targeting are glycosylated cell surface antigens that are expressed on solid tumors, such as carcinoembryonic antigen (CEA). CEA represents an attractive antigenic target for several reasons. It is a tumor-associated antigen that it is absent or poorly expressed by normal tissues and highly expressed by the vast majority of carcinomas of breast, colon, lung, pancreatic, ovarian, and medullary thyroid origin. High mortality rates coupled with suboptimal diagnostic and therapeutic options for these malignancies result in a serious, persistent public health problem. A fusion protein of Onconase comprising RNase from Rana pipiens -LL2-scFv are described herein.

CEA is a glycosylated cell surface protein of approximately 180 kDa, and is a solid tumor antigen that has been extensively studied clinically, both as a circulating tumor marker and as an antigenic target for radiolabeled mAbs for imaging and therapy. A number of anti-CEA antibodies have been under study in phase I-III clinical diagnostic and therapeutic trials. Exemplary of an anti-CEA mAb is the MN14 mAb. A humanized version of this mAb, hMN-14, in which human constant and framework regions replace the corresponding mouse sequences, has been constructed and expressed and is the mAb used in these clinical trials. A ^{99m}Tc-labeled Fab' fragment of another, related anti-CEA mAb, Immu-4, is useful in the detection and staging of colon cancer.

Other exemplary antigens for targeting include different B-cell restricted CD (clusters of differentiation) antigens, including CDs19-22, CD37, and HLA-DR. Preferred antigens are CD20, which is expressed at a high antigen density in a wide range of B-cell malignancies, ranging from acute lymphocytic leukemia (ALL) to the more differentiated B-Cell (B-CLL) and non-Hodgkin's lymphoma (NHL), and even to hairy cell leukemia (HCL), and CD22, an efficiently internalizing antigen which is associated with virtually all non-Hodgkin's lymphomas.

An antibody for targeting Onconase to Non-Hodgkins lymphoma is LL2 (IgG2a/kappa), a murine monoclonal antibody. Results show that LL2 is rapidly internalized after cell surface binding, with dimeric IgG or F(ab')₂ exhibiting a faster rate than monomeric Fab'. The antibody appears to be degraded mainly in lysosomes, since degradation is inhibited significantly in the presence of lysosomal inhibitors such as ammonium chloride or leupeptin.

VK and VH sequences for LL2 can be cloned using PCR-amplification, using the method and primers described by Orlandi *et al*., *PNAS*, **86**: 3833 (1989).

Suitable antibody fragments to these antigens include F(ab')₂, F(ab)₂, Fab', Fab, Fv and the like, including hybrid fragments. Also useful are any subfragments that retain the hypervariable, antigen-binding region of an immunoglobulin, including genetically-engineered and/or recombinant proteins, whether single-chain or multiple-chain, which incorporate an antigen binding site and otherwise function *in vivo* as targeting moieties in substantially the same way as natural immunoglobulin fragments.

Single-chain binding molecules are disclosed in U.S. Patent 4,946,778. Fab' antibody fragments may be conveniently made by reductive cleavage of F(ab')₂ fragments, which themselves may be made by pepsin digestion of intact immunoglobulin. Fab antibody fragments may be made by papain digestion of intact immunoglobulin, under reducing conditions, or by cleavage of F(ab)₂ fragments which result from careful papain digestion of whole Ig. The fragments may also be produced by genetic engineering.

Cytokine receptors such as IL-1R, IL-2R, IL-4R, IL-6R, IL-7R, IL-9R, IL-13R and IL-15R also can be targeted by targeting moieties. In one embodiment, cytokine receptors can targeted to the surface of cells that normally lack such receptors by the use of mAb-receptor conjugates, as described in copending application Serial No. 08/949,758, filed October 14, 1997. Receptors for growth factors like insulin and epidermal growth factor (EGF) can also be used to target rOnconase to a specific cell type.

Fusion proteins including Rana pipiens RNAse -LL2-scFv according to the invention can be produced recombinantly, using the same basic methodology described above. For example, cDNA that encodes NfM-onconase can be inserted into a plasmid that also contains cDNA that encodes a single-chain antibody fragment (scFv). Because the N-terminal pyroglutamyl residue is essential for the RNAase and cytotoxic activity of Onconase, the NfM-Onconase cDNA is inserted so that the expression product is [NfM-Onconase]-[scFv]. Recombinantly-produced fusion proteins can be purified, the N-formyl-methionine removed and the terminal glutaminyl residue cyclized to produce pyroglutamate as described above for recombinantly-produced Onconase.

Recombinantly-produced 'Onconase fusion proteins according to the invention are formulated into pharmaceutical compositions for treating lymphoma. In this context, the compositions comprise a solution of the Onconase fusion protein dissolved in a pharmaceutically acceptable carrier, preferably an aqueous carrier such as buffered saline. These solutions are sterile and may contain auxiliary substances such as pH adjusting and buffering agents and toxicity adjusting agents.

Dosage of Onconase molecule fusion protein according to the invention is about 0.1 to 10 mg per patient per day, although dosages of up to 100 mg per patient per day may be used, particularly when the drug is administered locally, and not into the bloodstream. Like native Onconase, recombinantly-produced Onconase according to the invention is readily internalized in cells, has anti-tumor effects *in vivo*, and preferentially kills rapidly dividing tumor cells. Chemical conjugates and fusion proteins provide for more specific targeting of the recombinantly-produced Onconase to particular cells.

In therapeutic applications, compositions are administered to a patient suffering from a disease, in a cytotoxic amount, which is defined as an amount sufficient to kill cells of interest. An amount successful to accomplish this is defined as a "therapeutically effective amount." The exact amount will depend on the severity of the disease and the general state of the patient's health. Single or multiple administrations of the compositions may be administered depending on the dosage required.

Onconase fusion proteins according to the invention can also be used to treat populations of cells *in vitro*. For example, it may be used selectively to kill unwanted cell types in bone marrow prior to transplantation into a patient undergoing marrow ablation.

The following examples are illustrative of the present invention, but are not to be construed as limiting.

### Example 1. Synthesis of PCR-amplified DNA encoding NfM-onconase

A 139-mer DNA nucleotide, ONCO-N, with the sense strand sequence [5'-TGG CTA ACG TTT CAG AAG AAA CAT ATC ACG AAT ACA CGA GAT GTA GAC TGG GAC AAT ATA ATG TCT ACG AAT CTG TTT CAC TGT AAG GAT AAG AAT ACC TTT ATA TAC AGT CGG CCA GAG CCT GTA AAG GCT ATC TGT A-3'] encoding an N-terminal sequence (46 amino acids) of recombinant Onconase is synthesized by an automated DNA synthesizer (Applied Biosystem 392 DNA/RNA Synthesizer) and used as the template for PCR-amplification with the flanking primers ONNBACK [5'-AAG CTT CAT ATG CAG GAT TGG CTA ACG TTT CAG AAG AAA-3', and ONNFOR [5'-CTT ACT CGC GAT AAT GCC TTT ACA GAT AGC CTT TAC AGG CTC TG-3']. The resultant double-stranded PCR product contains cDNA sequence that encodes for 54 amino acid residues of the N-terminal half of Onconase. ONNBACK contains the restriction sites HindIII (AAAGCTT) and NdeI (CATATG) to facilitate subcloning into either a staging vector or for in-frame ligation (NdeI site) into the bacterial expression vector. The NruI site (TCGCGA) is incorporated in the ONNFOR primer to facilitate in-frame ligation with the cDNA encoding the C-terminal half of Onconase.

Similarly, a 137-mer DNA nucleotide, ONCO-C, with the sense-strand sequence [TGC TGA CTA CTT CCG AGT TCT ATC TGT CCG ATT GCA ATG TGA CTT CAC GGC CCT GCA AAT ATA AGC TGA AGA AAA GCA CTA ACA AAT TTT GCG TAA CTT GCG AGA ACC AGG CTC CTG TAC ATT TCG TTG GAG TCG GG-3'] encoding the C-terminal sequence (46 amino acids) of Onconase is synthesized and PCR-amplified by the primers ONCBACK[5'-ATT ATC GCG AGT AAG AAC GTG CTG ACT ACT TCC GAG TTC TAT-3'] and ONCFOR[5'-TTA GGA TCC *TTA* GCA GCT CCC GAC TCC AAC GAA ATG TAC-3']. The final double-stranded PCR product contained a cDNA sequence that encoded 51 amino acids of the rest of the C-terminal half of Onconase. A NruI site allowed in-frame ligation with the N-terminal half of the PCR-amplified DNA incorporated in ONCBACK. A stop codon (shown in bold italics) and BamHI restriction sites (underlined) for subcloning into staging or expression vectors were included in the ONCFOR sequence.

The PCR-amplified DNA encoding the N- and C-terminal half of NfM-Onconase, after being treated with the appropriate restriction enzymes, were joined at the NruI sites and subcloned into a staging vector, *e.g*., pBluescript from Stratagene. The ligated sequence should encode a polypeptide of 105 amino acids with an N-terminal Met.

### Example 2. Cloning of LL2 and MN14 V-region sequences and humanization of LL2 and MN14

The V-region sequences of hLL2 and hMN14 have been published. Leung *et al*., *Mol. Immunol*., **32**:1413 (1995); U.S. 5,874,540. The VK and VH sequences for LL2 and MN14 were PCR-amplified using published methods and primers.

Sequence analysis of the PCR-amplified DNAs indicated that they encoded proteins typical of antibody VK and VH domains. A chimeric antibody constructed based on the PCR-amplified LL2 and MN14 sequences exhibited immunoreactivity comparable to their parent antibodies, confirming the authenticity of the sequence obtained.

Sequence analysis of the LL2 antibody revealed the presence of a VK-appended N-linked glycosylation site in the framework-1 region. Mutational studies indicated that glycosylation at the VK-appended site was not required to maintain the immunoreactivity of the antibody. Without the inclusion of the FR-1 glycosylation site, REI framework sequences were used as the scaffold for grafting the light chain CDRs, and EU/NEWM for grafting the heavy chain CDRs of LL2. The immunoreactivity of the humanized LL2 (hLL2) was shown to be comparable to that of murine and chimeric LL2. The rate of internalization for LL2 was not affected by chimerization or humanization of the antibody.

### Example 3. Construction of gene encoding fusion protein of humanized LL2 and NfM-onconase

The VH and VK sequences of hLL2 were used as templates to assemble the hLL2-scFv gene by standard PCR procedures. The configuration of the gene was Met(-1)-VL-(GGGS)₄-VH-(His)₆. A Met (ATG) initiation codon at the -1 position was incorporated at the N-terminus of the VL gene, which was linked via a 16 amino acid linker (GGGS)₆ to the VH domain. A tail consisting of six histidyl residues was included at the carboxyl end of the VH chain to facilitate the purification of the fusion protein via metal chelate chromatography.

The immunotoxin fusion protein gene for NfM-Onconase-hLL2scFv was constructed in a similar fashion by restriction digestion and ligation methods. THe cDNA sequence, when expressed, encoded a fusion protein of the structure:
NfM-Onconase-[linker]-VL-(GGGS)₄-VH-(His)₆.

There are a variety of linkers that can be inserted between the NfM-Onconase C-terminus and the VL domain N-terminus. A preferable linker is the amino acid sequence TRHRQPRGW from the C-terminal position 273-281 of *Pseudomonas* exotoxin (PE). This sequence has been shown to be a recognition site for intracellular cleavage of PE into active fragments by subtilisins, with cleavage occurring between the G and W residues of the sequence. Chiron *et al., J. Biol Chem.,* **269**:18167 (1994). Incorporation of this sequence facilitates the release of active rOnconase after internalization of the fusion immunotoxin. Alternatively, a 13-amino acid residue spacer consisting of amino acid residues 48-60 of fragment B of *Staphylococcal* Protein A, used in the construction of an EDN-scFv fusion, can be used instead to allow for flexible linkage between the rOnconase and the scFv. Tai *et al*., *Biochemistry,* **29**:8024 (1990) and Rybak *et al*., *Tumor Targeting*, **1**:141 (1995).

### Example 4. Construction of gene encoding fusion protein of humanized MN14 and NfM-Onconase

MN14 scFᵥ was produced by PCR amplification of cDNA from humanized MN14 transfectoma. The linker used for MN14 scFᵥ was a 15-amino acid linker (GGSGS)₃ and the orientation was V_{L}-linker-V_{H}. After confirmation of the DNA sequences, the single chain construct was subcloned into an appropriately restricted expression plasmid used for other scFᵥs. This construct then was transformed into BL21(λDE3) *E. coli* for expression.

Another single chain construct also was made. This was made with the opposite 5'-3' orientation of the heavy and light chains, was assembled in pCANTABESE (Pharmacia Biotech, Piscataway, NJ) and expressed in phage. Specific binding of recombinant phage expressing this scFᵥ was demonstrated by ELISA.

The V_{L}-linker-V_{H} sequence was used for construction of Onconase-MN14 fusion protein, as diagrammed below. The DNA fragment encoding Onconase was obtained according to Example 1. A 23-amino acid linker was used between the Onconase sequence and the scFᵥ. Kurucz *et al.* (1995). Alternatively, the (GGSGS)₃ linker which was used in construction of the MN14 scFᵥ described above was used. A preferred configuration of the fusion protein was:
**Onconase -linker--V**_{**L**}**―(GGSGS)**_{**3**}**--V**_{**H**}

### Example 5. Expression and purification of hLL2-scFv, hMN14-scFv, NfM-Onconase-hLL2-scFv immunotoxin, and NfM-Onconase-hMN14-scFv immunotoxin

A vector for expression of hMN14-scFv and hLL2-scFv is the commercially-available T7 promoter-driven pET vector (Novagen, Madison, WI). The DNA expression vectors scFvhMN14-pET and scFvhLL2-pET are derived from pET and contain the scFv sequence for hMN14 or hLL2 fused to PE40 which was destroyed by a SalI/XhoI deletion, with a T7 promoter.

NfM-Onconase cDNA was digested with NdeI/BamHI and cloned into the corresponding sites in the vectors scFvhMN14-pET and scFvhLL2-pET, and the sequences were confirmed. A redundant sequence between the BamHI and the EagI site within the expression vector scFvhMN14-pET was removed by restriction digestion, gel-purification and religation. The fragment removed was found to contain part of the scFvMN14 and the defective PE40 gene. The final scFvhMN14-NfM-Onconase expression vector was further sequenced and designated rOnpET.

Large scale expression of the recombinant protein from the T7-driven rOnpET vector requires an appropriate host *E. coli*, such as BL21, which contains a λDE3 lysogen, as described above. rOnpET vector was used to transform competent BL21/λDE3 cells by electroporation. Colonies that survived selection on Amp agar plates were picked and grown in a shaker incubator at 37°C in 3 ml of LB-Amp. After incubation for 8-10 hours, 100 µl of the culture was tranferred to 25 ml of superbroth (LB supplemented with 0.5% glucose, 1.6 mM MgSO₄ and 100 µg/ml ampicillin) in a 500 ml E-flask to increase aeration while shaking. The culture was incubated overnight in the shaker incubator at 37°C. The culture then was transferred into one liter of superbroth and further incubated in a shaker incubator at 37°C. IPTG at a final concentration of 1 mM was added into the culture when the OD650 of the culture reached 1 (approx. 2.5 hours). Induction was allowed to proceed for 1-3 hours before the culture was terminated for inclusion body isolation. Ten µl of the culture was analyzed under reducing conditions in 15% SDS-PAGE gel. Colonies with the highest level of induction were kept as stock culture and stored frozen at -70°C.

The λDE3 lysogen carries the inducible T7 RNA polymerase gene. The results showed that expression of the T7 polymerase was induced by the addition of IPTG, and the expressed T7 polymerase in turn activated the transcription of the T7-driven recombinant protein. T7 promoter-driven transcription was so efficient that the protein was abundantly expressed and precipitated in the cytoplasm as inclusion bodies.

Inclusion body isolation entailed lysis of cells by homogenization in the presence of lysozymes to release the incudions bodies as insoluble pellets. The washed inclusion bodies were dissolved in denaturing buffer that contained 7 M of guanidine-HCl. Disulfide bonds were reduced by dithioerythritol and then were refolded by dropwise dilution of the denatured protein in renaturing buffer that contained arginine-HCL and oxidized glutathione.

### Example 6. Expression and purification of NfM-Onconase

A 6-liter culture equivalent of renatured inclusion bodies from Example 5 was purified. Harvested cell paste was resuspended using a Tisuemizer tip (Thomas, Swedesboro, NJ) in TES buffer (50 mM Tris, pH 8, 100 mM NaCl, and 20 mM EDTA) containing 180 µg/ml lysozyme. After incubating at 22°C for one hours, the cells were resuspended again and centrifuged at 27,000g for 50 minutes. The pellet was washed by resuspension and centrifugation three or four times with TES buffer containing 2.5% Triton-X-100 and then four times with TES. The inclusion bodies were resuspended in 5 to 10 ml of denaturation buffer (7 M guanidine:HCl, 0.1 M Tris, pH 8.0, and 5 mM EDTA) by sonication or tissuemizing and diluted to a protein concentration of 10 mg/ml.

The protein was reduced with dithioerythritol (65 mM) for 4 to 24 hours at 22°C and rapidly diluted in a thin stream into refolding buffer (0.1 M Tris, pH 8.0, 0.5 arginine:HCl, 2 mM EDTA, and 0.9 mM oxidized glutathione). After incubating at 10°C for 36 to 72 hours, the refolded NfM-Onconase was dialyzed against 0.15 M sodium acetate (pH 5), and was loaded onto a HiLoad 16/20 SP cation exchange FPLC column. After buffer exchange into 0.15 M sodium acetate (pH 5), precipatates formed. These were removed by centrifugation. Elution with a 0-1 M linear gradient of sodium chloride was used, and fractions corresponding to absorbance peaks were analyzed by SDS-PAGE (15%). Most column-bound proteins eluted at 0.5 M NaCl.

The eluted products were roughly divided into three fractions. Fraction I constituted the major peak. Fractions II and III were relatively minor peaks which eluted in advance of fraction I, with fraction II being a shoulder peak of fraction I.

### Example 7: Removal of Met from NfM-Onconase and NfM-Onconase fusion proteins

The N-terminal Met residues of NfM-Onconase, NfM-Onconase-hMN14-scFv and NfM-Onconase-hLL2-scFv were removed according to Shapiro *et al*. (1988). 100-200 µg/ml of purified and renatured proteins were incubated with 0.5 µg/ml *Aeromonas proteolytica* aminopeptidase (Sigma Chemicals, St. Louis, MO) in 200 mM sodium phosphate, pH 7.5 for 18 hours at 37°C.

### Example 8: Preparation of antibody rOnconase conjugates

Disulfide-linked conjugates were prepared as described by Newton *et al*., *J. Biol*. *Chem*., **267**: 19572 (1992), with some modification. 2-Iminothiolane-modified antibody was incubated overnight at 23°C with a 40-fold excess of rOnconase modified with N-succinimidyl-3-(2-pyridyldithio)-proprionate (SPDP) (1.1-1.3 mol SPDP/mol rOnconase). Rybak *et al*., *J. Biol. Chem*., **266**: 21202 (1991). Thioether-linked conjugates were prepared according to standard procedures, using *m*-maleimidobenzoyl-N-hydroxylsuccinimide ester (MBS). Gulberg *et al., Biochem. Biophys. Res. Commun.,* **139**: 1239 (1986). Two separate tubes containing 2 mg of antibody were incubated with a 5-fold molar excess of MBS (stock solution, 30 mM in dimethylformamide) for 10 minutes at 23°C. The tubes were pooled and applied to a PD 10 column equilibrated with buffer A (0.1 M NaPO₄, pH 7.5, containing 0.1 M NaCl). THe peak fractions, each containing 1.5 ml, were pooled. Buffer A was exchanged into buffer B (0.1 M Na acetate, pH 4.5, containing 0.1 M NaCl) by repeated concentration followed by reconstitution of the recovered retentate with buffer B using Centricon 10 microconcentrators (Amicon). The SPDP-modified rOnconase was reduced for 30 minutes at 23°C with 23 mM dithiothreitol and gel filtered on a PD 10 column equilibrated with buffer A. The peak fractions, about 1.5 ml each, were added to tubes containing 750 µl of MBS-modified antibody and incubated overnight at 23°C. The next day conjugates were incubated with 0.5 mM N-ethylmaleimide (20 mM) in dimethylformamide for at least one hour before purification by size-exclusion high-performance liquid chromatography on a TSK 3000 column (Toso Haas Corp., PA) equilibrated and eluted with 0.1 M phosphate buffer, pH 7.4. The flow rate was 0.5 ml/min and 1-minute fractions were collected. Peak fractions were pooled and analyzed by SDS-PAGE to determine the amount of free ligand and rOnconase in the conjugates.

### Example 9: In vitro activity of rOnconase and rOnconase immunotoxins

The ability of rOnconase to inhibit protein synthesis in a rabbit reticulocyte lysate was assessed using protocols described by St. Clair *et al*., *PNAS USA,* **84**: 8330 (1987). All fractions were passed through Superose 12 FPLC before being tested for activity, however, because aminopeptidase has a molecular weight of 29 kD and NfM-Onconase has a molecular weight of 12 kD, the two could not be separated by size exclusion chromatography using Superose 12 FPLC. Nevertheless, results confirmed that rOnconase inhibited protein synthesis.

Native Onconase (AlfaCell), fraction I untreated with aminopeptidase (negative control), and fractions I, II and III treated with aminopeptidase (AP) at different molar concentrations were added to the *in vitro* translation system. ³²Met incorporation was measured after incubating the mixture with or without ribonucleases in a scintillation counter, to assess the rate of protein synthesis. Protein concentrations of different samles were determined at the same time by BCA assay (Pierce) using BSA as the standard.

Results showed that fraction I (AP) and Onconase (AlfaCell) exhibitied identical RNase activities. Fraction II (AP) and fraction III (AP) also exhibited RNase activity, but at a lower potency, indicating that these fractions comprise rOnconase that has not refolded properly. The activity in the fractions II (AP) and III (AP) likely was a spill over from fraction I (AP). Fraction I (negative control) exhibited substantially lower activity than fraction I (AP). Addition of an excess of aminopeptidase to the translation system did not change the results, demonstrating that the lower activity of fraction I as compared to fraction I (AP) was not due to incomplete removal of aminopeptidase following removal of the N-terminal Met, and that it was the N-terminal Met that was inhibiting RNase activity.

The ability of rOnconase to inhibit protein synthesis by three B-lymphoma cell lines, Daudi, Raji, and CA-46, and a human T cell line, Hut 102 also was assessed. Cells were plated at concentrations of 2 x 10⁵ cells/ml in 96-well microtiter plates overnight in the appropriate complete media. The complete media were replaced by serum-free and leucine-free media containing increasing concentrations of rOnconase and rOnconase immunotoxin, both recombinantly-produced and chemically-conjugated, for 16 hours followed by a 1 hour pulse with 0.1 µCi of [¹⁴C]leucine. Cells were harvested onto glass fiber filters using a cell harvester (Skaron), washed with water, dried with ethanol, and counted. Cytotoxic/cytostatic effects on the cells were assessed by a method that differed only in that a 1 hour pulse with 0.5 µCi of [³H] thymidine was used.

The results showed that rOnconase and recombinantly-produced and chemically-conjugated rOnconase immunotoxins all inhibited protein synthesis (IC₅₀ of 10-100 pM) and produced cytotoxic/cytostatic effects in human lymphoma cells. The *in vitro* cyctotoxicity of the immumoconjugate was enhanced to IC₅₀ 0.9 pM by monensin. By comparison, conjugates of LL2 with either EDN (eosinophil RNase) or pancreatic RNase A (hpanc) were considerably less cytotoxic, with IC₅₀ of hLL2-EDN of 70 nM and that of hLL2-hpanc greater than 50 nM, respectively. The cytotoxicity of LL-2 conjugates versus their component proteins are summarized in Table 1.

**Table 1**

| | IC₅₀ (pM) | | | |
|---|---|---|---|---|
| | LL2-rOnconase | rOnconase | LL2 | LL2-EDN |
| Daudi | 50-500 | >200,000 | >23,000 | >43,000 |
| CA-46 | 800 | >200,000 | >23,000 | >43,000 |
| Raji | 800 | >200,000 | >23,000 | |
| Hut-102 | >40,000 | 37,000 | | |

Conjugates of hLL2-rOnconase display similar results with an IC₅₀ or 400-500 pM when tested on Daudi cells.

### Example 10: In vivo activity of rOnconase and rOnconase immunotoxins

SCID mice with minimal disease and with more advanced Daudi lymphoma were treated with LL2-rOnconase (100 µg QD x5). The mice enhibited increased life span of 216% and 135%, respectively.

## Claims

1. A recombinantly-expressed fusion protein comprising a molecule having the sequence of RNAse from *Rana pipiens* fused to the amino terminal of the light chain of a single-chain antibody fragment (scFv) wherein said RNAse molecule has pyroglutamate as the N-terminal residue and said scFv is an LL2 scFv.

2. A fusion protein according to claim 1 having the structure RNAse-linker-VL-linkerVH.

3. A fusion protein according to claim 2, wherein the LL2 scFv is humanised.

4. A composition comprising a fusion protein according to any preceding claim and a pharmaceutically acceptable carrier.

5. Use of a recombinantly-expressed fusion protein according to claim 1 or claim 2 in the preparation of a medicament for the treatment of lymphoma.

## Patentansprüche

1. Rekombinant exprimiertes Fusionsprotein, das ein Molekül umfaßt, welches die Sequenz von RNAse von *Rana pipiens* aufweist, das mit dem Aminoterminus der leichten Kette eines Einzelkettenantikörperfragments (scFv) fusioniert ist, wobei das RNAse-Molekül Pyroglutamat als den N-terminalen Rest aufweist und das scFv ein LL2-scFv ist.

2. Fusionsprotein nach Anspruch 1, welches die Struktur RNAse-Linker-VL-Linker-VH aufweist.

3. Fusionsprotein nach Anspruch 2, wobei das LL2-scFv humanisiert ist.

4. Zusammensetzung, die ein Fusionsprotein nach einem der vorangegangenen Ansprüche und einen pharmazeutisch verträglichen Träger umfaßt.

5. Verwendung eines rekombinant exprimierten Fusionsproteins nach Anspruch 1 oder Anspruch 2 bei der Herstellung eines Medikaments für die Behandlung von Lymphom.

## Revendications

1. Protéine de fusion exprimée par recombinaison, comprenant une molécule ayant la séquence de RNAse provenant de *Rana pipiens* fusionnée à l'extrémité amino-terminale de la chaîne légère d'un fragment d'anticorps monocaténaire (scFv), dans laquelle ladite molécule de RNAse possède un pyroglutamate comme résidu N-terminal, et ledit scFv est un scFv LL2.

2. Protéine de fusion suivant la revendication 1, ayant la structure RNAse-segment de liaison-VL-segment de liaison-VH.

3. Protéine de fusion suivant la revendication 2, dans laquelle le scFv LL2 est humanisé.

4. Composition comprenant une protéine de fusion suivant l'une quelconque des revendications précédentes et un support pharmaceutiquement acceptable.

5. Utilisation d'une protéine de fusion exprimée par recombinaison suivant la revendication 1 ou la revendication 2, dans la préparation d'un médicament destiné au traitement d'un lymphome.
